Europäisches Patentamt

European Patent Office

Office européen des brevets

(11) Publication number: **0 137 173**
**B1**

(12) **EUROPEAN PATENT SPECIFICATION**

(45) Date of publication of patent specification: 25.04.90

(51) Int. Cl.⁵: **A 61 K 7/32**

(21) Application number: 84109095.4

(22) Date of filing: 01.08.84

(54) Deodorant formulations.

(30) Priority: 11.08.83 GB 8321654

(43) Date of publication of application:
17.04.85 Bulletin 85/16

(45) Publication of the grant of the patent:
25.04.90 Bulletin 90/17

(84) Designated Contracting States:
AT BE CH DE FR GB IT LI LU NL SE

(56) References cited:
DE-A-2 354 517
FR-A-2 339 395
FR-A-2 518 878
GB-A-1 465 665
US-A-4 035 510

SEIFEN-ÖLE-FETTE WACHSE, vol. 98, no. 16, 3rd
August 1972, page 511; "Antworten"

SEIFEN-ÖLE-FETTE-WACHSE: KOSMETIK UND
AEROSOLE, vol. 99, no. 6/7, 21st March 1973,
pages 155-159; E.L. ROEHL: "Die kosmetischen
Deodorant- und Antiperspirant-Präparate"

(73) Proprietor: BEECHAM GROUP PLC
Beecham House Great West Road
Brentford Middlesex TW8 9BD (GB)

(72) Inventor: Graham, June
51 Lemsford Lane
Welwyn Garden City Hertfordshire AL8 6YN (GB)

(74) Representative: Russell, Brian John et al
Beecham Pharmaceuticals
Great Burgh
Yew Tree Bottom Road
Epsom Surrey KT18 5XQ (GB)

EP 0 137 173 B1

# EP 0 137 173 B1

**Description**

The present invention relates to quick-drying deodorant formulations.

In order to produce quick-drying formulations it is customary to use an aqueous ethanol base due to its high volatility. However typical alkaline deodorant agents such as sodium bicarbonate are not soluble in ethanol and must therefore be produced as opaque gels or suspensions. Active ingredients which are soluble in ethanol, such as antibacterials, can only prevent the formation of malodour and sorbants such as zinc ricinoleate, whilst reducing existing malodour also tend to absorb the perfume which is usually included in deodorant formulations.

German Offenlegungsschrift DE—A—2354517 discloses a formulation comprising an antimicrobial lichen acid action as a deodorant agent, a hydroxyamino compound acting as solubilizer, and ethanol.

FR—A—2 339 395 discloses a deodorant composition containing a mixture of a weak organic acid and a salt of a weak organic acid with an animated organic compound such as aminoalcohol. FR—A—2 518 878 discloses a liquid deodorant composition containing propylene glycol, a dye, and, as solubilizer triethamolamine.

It has now surprisingly been discovered that certain amine alcohols can be employed as deodorant agents and, because they are soluble in aqueous alcohol bases, it is possible to produce clear formulations and so to avoid the difficulties of providing stable suspensions. Such amino alcohols bot only suppress the formation of axillary malodour but can also combat existing malodour.

Accordingly the present invention provides a deodorant formation comprising: a lower alcohol having up to six carbon atoms; from 0.2 to 2% w/w of an amino-lower or -middle alkanol having-up to 10 carbon atoms in a straight or branched chain bearing from one to three hydroxy groups and at least one amino group on a carbon atom not bearing a hydroxy group, wherein the amino group is optionally substituted with one $C_{1-6}$ alkyl group; and from 0 to 60% of water, characterized in that the amino-lower or- middle alkanol alone acts as an active deodorant ingredient, and with the proviso that a lichen acid is not present, and that the amino-lower or -middle alkanol is not triethenolamine.

A particularly suitable amino alkanol is 2-amino-2-methylpropan-1-ol.

Preferably the formulation contains from 0.5 to 1% w/w of the amino alkanol.

The exact proportion of lower alcohol in the formulation will depend on the type of presentation. The alcohol content will not usually exceed 70% by weight, the lower limit will generally be about 15%.

Preferably the lower alcohol is ethanol.

Preferably the formulation contains from 20 to 40% w/w of water.

The formulation may be presented in any conventional form, such as roll-on, spray, aerosol, or stick formulation, or it may be presented as impregnated moist tissues. Conventional accessory ingredients such as thickeners, perfumes and preservatives may be included as appropriate to the presentation. Thus a roll-on formulation may include a thickener such as hydroxypropyl cellulose whereas an aerosol formualtion would not require any thickener.

For aerosol formulations suitable propellants would be dichlorodifluoromethane, or a mixture of fluorocarbons and/or hydrocarbons, such as n-butane, isobutane and *n*-propane.

For stick formulations, the formulation typically also includes a soap, such as sodium stearate. The molten product is run into a mould to form a stick.

When presented as moist tissues, about $5cm^3$ of the formulation is applied to each crepe tissue about $20cm^2$ in area. The tissues are then individually packaged in sealed sachets. Suitable accessory ingredients for a formulation to be applied to such tissues include emollients, perfumes and preservatives.

Other conventional ingredients may also be included such as compatible antiperspirants and/or antimicrobial agents. One suitable antimicrobial agent is Chlorhexidine [1,6-di-(N-chlorophenyldiquanido)-hexane] which may be included in the formulation at from 0.2 to 4%, preferably about 1% w/w. A preferred antimicrobial agent however is Irgasan DP 300 (Triclosan or 2, 4, 4-trichloro- 2-hydroxydiphenylether) included at from 0.1 to 1% w/w.

Formulations according to the invention may be produced by conventional methods. Typically the amino alkanol is dissolved in the alcohol and then water is added as required. The formulations are then filled into conventional dispensers, such as roll-on dispensers, spray pump packs or aerosol dispensers, holders for a self-supporting stick compositions, or impregnated into tissues.

2

# EP 0 137 173 B1

The invention will now be illustrated with reference to the following Examples:

## Example 1

### Aqueous alcoholic roll-on

|  | % w/w |
| --- | --- |
| De-ionized water | 37.8% |
| Hydroxypropyl cellulose | 0.7% |
| Amino-methyl-propanol | 1.0% |
| Alcohol | 60.0% |
| Perfume | 0.5% |

### Procedure

The hydroxypropyl cellulose is dissolved in part of the alcohol with a high shear stirrer and allowed to solvate. The amino methyl propanol and perfume are dissolved in the remaining alcohol and the mixture is added to the batch. Water and the other ingredients are added slowly with stirring.

## Example 2

### Aerosol Deodorants

|  | % w/w |
| --- | --- |
| Amino-methyl-propanol | 1.0% |
| Irgasan DP 300 | 0.1% |
| Perfume | 0.5% |
| Alcohol | 50.0% |
| Propellant 12 | 48.4% |

(Propellant 12 is dichlorodifluoromethane). The composition is produced conventionally and filled into aerosol spray cans.

### Clinical Trial

#### Deodorant

|  | A (test product) | B (control product) |
| --- | --- | --- |
| Amino-methyl-propanol | 1.00% | — |
| Alcohol | 60.00% | 60.00% |
| Water | 39.00% | 40.00% |

14 male volunteers first used soap free of antimicrobials in their axillae, for a period of 8 days; no deodorants or antiperspirants were applied. One day 8 each volunteer had the malodour in their axillas assessed by 4 trained observers. One half of the group had deodorant A applied to their right axilla and deodorant B applied to their left axilla whilst the other half had A applied on the left and B applied on the right. Axillary malodour was then reassessed by the observers after the products had dried, then 3½, 5½ and 24 hours later. The scores recorded by each observer for each subject were combined to give 5 mean scores for deodorants A and B.

Mean percentage changes in odour scores were calculated by comparing the mean score for the active

3

treated (deodorant A) and the control treated (deodorant B) axilla with the control ratio before treatment: % change in malodour, × hours after treatment =

$$100 \left\{ A \frac{A}{BC} - 1 \right\}$$

A = mean score for axilla treated with deodorants A, × hours after treatment.
B = mean score for axilla treated with deodorant B, × hours after treatment.

$$C = \text{control ratio} = \frac{\text{mean score for axilla before treatment with deodorant A}}{\text{mean score for axilla before treatment with deodorant B}}$$

Immediately after application deodorant A reduced the mean malodour score by 62% compared with deodorant B (p <0.01). After 3½ and 5½ hours, 27% and 33% reductions in axillary malodour were obtained with deodorant A compared to deodorant B.

## Claims

1. A deodorant formulation comprising: a lower alcohol having up to six carbon atoms; from 0.2 to 2% w/w of an amino-lower or -middle alkanol having up to 10 carbon atoms in a straight or branched chain bearing from one to three hydroxy groups and at least one amino group on a carbon atom not bearing a hydroxy group, wherein the amino group is optionally substituted with one $C_{1-6}$ alkyl group; and from 0 to 60% of water, characterised in that the amino-lower or-middle alkanol alone acts as an active deodorant ingredient, and with the proviso that a lichen acid is not present, and that the amino-lower or -middle alkanol is not triethanolamine.

2. A deodorant formulation according to claim 1 wherein the amino alkanol is 2-amino-2-methyl-propan-1-ol.

3. A deodorant formulation according to claim 1 or 2, wherein the formulation contains from 0.5—1% w/w of the amino-lower or -middle alkanol.

4. A deodorant formulation according to any one of claims 1 to 3 wherein the formulation contains from 15 to 70 % w/w of the lower alcohol having up to six carbon atoms.

5. A deodorant formulation according to any one of claims 1 to 4 wherein the alcohol is ethanol.

6. A deodorant formulation according to any one of claims 1 to 5 wherein the formulation comprises from 20 to 40% w/w of water.

7. A deodorant formulation according to any one of claims 1 to 6 also comprising an antimicrobial agent.

8. A deodorant formulation according to claim 7 wherein the antimicrobial agent is 0.1 to 1% w/w of 2,4 4-trichloro-2-hydroxydiphenylether.

9. A method of deodorising the human body comprising the application of a deodorant formulation according to any one of claims 1 to 8.

## Patentansprüche

1. Eine Deodorant-Formulierung umfassend: einen niederen Alkohol mit bis zu 6 Kohlenstoffatomen; 0,2 bis 2 Gewichtprozent eines niederen oder mittleren Aminoalkanoles mit bis zu 10 Kohlenstoffatomen in einer geraden oder verzweigten Kette mit 1 bis 3 Hydroxygruppen und mindestens einer Aminogruppe an einem Kohlenstoffatomen, das keine Hydroxygruppe trägt, wobei die Aminogruppe gegebenenfalls mit einer $C_{1-6}$-Alkylgruppe substituiert ist; und 0 bis 60% Wasser, dadurch gekennzeichnet, daß das niedere oder mittlere Aminoalkanol als ein aktiver Deodorantbestandteil wirkt, und mit der Maßgabe, daß keine Flechtensäure (lichen acid) vorhanden ist und daß das niedere oder mittlere Amino-alkanol nicht Triäthanolamin ist.

2. Eine Deodorant-Formulierung nach Anspruch 1, in welcher das Aminoalkanol 2-Amino-2-methyl-propan-1-ol ist.

3. Eine Deodorant-Formulierung nach Anspruch 1 oder 2, in welcher die Formulierung 0,5 bis 1 Gewichtsprozent des niederen oder mittleren Aminoalkanols enthält.

4. Eine Deodorant-Formulierung nach einem der Ansprüche 1 bis 3, in welcher die Formulierung 15 bis 70 Gewichtsprozent des niederen Alkanols mit bis zu 6 Kohlenstoffatomen enthält.

5. Eine Deodorant-Formulierung nach einem der Ansprüche 1 bis 4, in welcher der Alkohol Äthanol ist.

6. Eine Deodorant-Formulierung nach einem der Ansprüche 1 bis 5, in welcher die Formulierung 20 bis 40 Gewichtsprozent Wasser enthält.

7. Eine Deodorant-Formulierung nach einem der Ansprüche 1 bis 6, welche auch einen antimikrobiellen Wirkstoff enthält.

8. Eine Deodorant-Formulierung nach Anspruch 7, in welcher der antimikrobielle Wirkstoff 0,1 bis 1 Gewichtsprozent des 2,4-4-Trichlor-2-hydroxydiphenyläthers ist.

9. Eine Methode zum Desordierendes menschlichen Körpers umfassend die Anwendung einer Deodorant-Formulierung nach einem der Ansprüche 1 bis 8.

## Revendications

1. Formulation déodorante, comprenant un alcool inférieur ayant jusqu'à six atomes de carbone, de 0,2 à 2% en poids d'un amino alcanol inférieur ou moyen ayant jusqu'à dix atomes de carbone dans une chaîne droite ou ramifiée portant un à trois groupes hydroxy et au moins un groupe amino sur une atome de carbone ne portant pas de groupe hydroxy, le groupe amino étant éventuellement substitué avec un groupe alkyl en $C_{1-6}$; et de 0 à 60% d'eau, caractérisé en ce que l'amino-alcanol inférieur ou moyen joue seul le rôle de composant déodorant actif, et à condition qu'un acide de lichen ne soit pas présent et que l'amino-alcool inférieur ou moyen ne soit pas la triéthanolamine.

2. Formulation déodorante suivant la revendication 1, caractérisée en ce que l'amino alcanol est le 2-amino-2-méthylpropan-1-ol.

3. Formulation déodorante suivant la revendication 1 ou 2, caractérisée en ce que la formulation contient de 0,5 à 1% en p/p de l'amino alcanol inférieur ou moyen.

4. Formulation déodorante suivant l'une quelconque des revendications 1 à 3, caractérisée en ce qu'elle contient de 15 à 70% en p/p de l'alcool inférieur ayant jusqua'à 6 atomes de carbone.

5. Formulation déodorante suivant l'une quelconque des revendications 1 à 4, caractérisée en ce que l'alcool est l'éthanol.

6. Formulation déodorante suivant l'une quelconque des revendications 1 à 5, caractérisée en ce qu'elle comprend 20 à 40 en p/p d'eau.

7. Formulation déodorante suivant l'une quelconque des revendications 1 à 6, caractérisée en ce qu'elle comprend aussi un agent antimicrobien.

8. Formulation déodorante suivant la revendication 7, caractérisée en ce que l'agent antimicrobien est constitué par 0,1 à 1% p/p d'éther 2,4,4-trichloro-2-hydroxy-diphénylique.

9. Méthode pour désodoriser le corps humain, caractérisée en ce qu'elle comprend l'application d'une formulation déodorante suivant l'une des revendications 1 à 8.